# EUROPEAN PATENT SPECIFICATION

(11) **EP 1 973 507 B1**
(45) Date of publication and mention of the grant of the patent: **20.04.2011**
(21) Application number: 07700700.3
(22) Date of filing: 04.01.2007
(51) Int. Cl.: A61F 9/00

(54) **INTRAOCULAR DRUG DISPENSER**
INTRAOKULARER ARZNEISPENDER
DISTRIBUTEUR DE MEDICAMENT INTRAOCULAIRE

(30) Priority: 17.01.2006 US 759060 P
(43) Date of publication of application: 01.10.2008
(73) Proprietor: Nulens Ltd, 46121 Herzliya (IL)
(72) Inventor: BEN NUN, Joshua, 40291 D.n.vitkin (IL)
(74) Representative: South, Nicholas Geoffrey
(86) International application number: PCT/IL2007/000007
(87) International publication number: WO 2007/083293

(56) References cited:
- WO-A-2005/016558
- WO-A-2005/065600
- DE-A1- 10 005 957
- US-A1- 2002 026 176
- US-A1- 2002 188 282

## Description

### Field of the Invention

The invention pertains to intraocular drug dispensers.

### Background of the Invention

Conventional intraocular drug dispensers include a drug reservoir from which a drug is released by diffusion or osmosis through a membrane for continuously administering drugs into an eye's vitreous cavity over relatively prolonged periods of time. However, the operation of such intraocular drug dispensers are often impeded by natural fibrous tissue growing thereon resulting in their actual drug delivery rate being lower than their intended drug delivery rate to the detriment of an intended treatment.

Other intraocular drug slow release devices are illustrated and described in *inter alia* US Patent 5,098,443 to Parel et al., US Patent 5,466,233 to Weiner et al., US Patent 5,830,173 to Avery et al., US Patent Application Publication No. US 2002/0110591 to Brubaker et al., and commonly assigned PCT International Publication No. WO 2005/065600.

US 2002/026176A1 discloses an intraocular drug dispenser having a plurality of compartments to be filled with different substances and having a rim or cap on one end which can be sutured to the sclera.

Certain eye conditions, for example, macular degeneration, require specific drug quantities at regular intervals, say, once a month, for unlimited periods of time. Shots of specific drug quantities are administered at the present time by eye injection notwithstanding that eye injections are highly traumatic to even healthy eyes and can lead to eye infections, and other undesirable conditions.

### Summary of the Invention

The present invention provides an Intraocular drug dispenser as claimed in claim 1.

The present invention is for intraocular drug dispensers intended for implantation in a circumferential band of an eye wall devoid of viable tissues called the pars plana. Implantation can be effected by a conventional three port vitrectomy. The intraocular drug dispensers include an elongated support member having a string of discrete drug containing capsules which are intended to individually release their contents into an eye's vitreous cavity. Drug containing capsules preferably have a biocompatible inert coating intended for rupturing by laser radiation. Alternately, drug containing capsules can have a biocompatible inert coating with a biodegradable plug for enabling time controlled drug release depending on thickness and/or rate of material degradation. The intraocular drug dispensers are preferably anchored in an eye's pars plana by T-shaped fixation members at each end of their support members. The T-shaped fixation members preferably include a pair of oppositely directed self-anchoring anchor members for drawing together opposite side walls of a throughgoing incision in accordance with afore-mentioned WO 2005/065600's Figure 5.

### Brief Description of the Drawings

In order to understand the invention and to see how it can be carried out in practice, preferred embodiments will now be described, by way of non-limiting examples only, with reference to the accompanying drawings in which similar parts are likewise numbered, and in which:
Fig. 1 is a schematic view of an intraocular drug dispenser for time controlled drug release in accordance with a first preferred embodiment of the present invention;
Fig. 2 is a schematic view of an intraocular drug dispenser for time controlled drug release in accordance with a second preferred embodiment of the present invention; and
Fig. 3 is a cross section of an eye implanted with Figure 1's intraocular drug dispenser for controlled intraocular drug release purposes.

### Detailed Description of Preferred Embodiments of the Present Invention

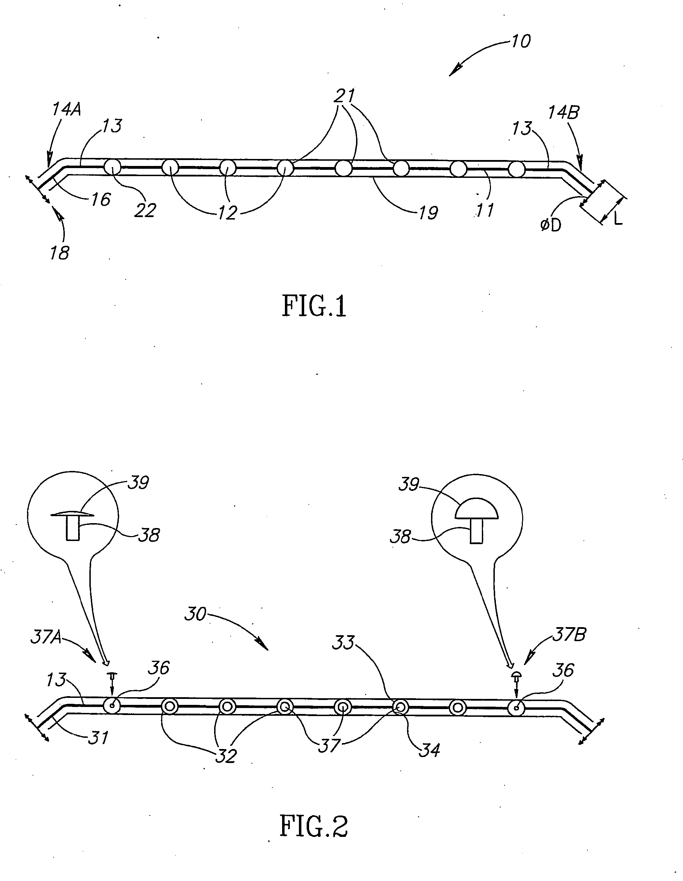

Figure 1 shows an intraocular drug dispenser 10 including an elongated support member 11 with a string of discrete drug containing capsules 12, and a pair of opposite ends 13. T-shaped fixture members 14A and 14B are mounted at the opposite ends 13 for securely mounting the intraocular drug dispenser 10 in an eye. The T-shaped fixture members 14A and 14B subtend an obtuse angle α≈170° with respect to the support member 11 for assisting in implanting the intraocular drug dispenser 10 in an eye. Each T-shaped fixture member 14 includes a strut 16 with a pair of oppositely directed self-anchoring elongated anchor members. The anchor members are formed with leading ends 18 which can be barbed, hooked, and the like, for anchoring into an eye wall. The drug containing capsules 12 can be enclosed by a soft silicone sheath 19 for protecting an eye's delicate internal structures on pulling the support member 11 thorough an eye from one vitrectomy port to another vitrectomy port.

The support member 11 has a total length in the region of 30mm±5mm such that the support member 11 can be urged into a horse shoe shape *in situ* to minimize visual disruption on implantation in an eye. The anchor members have a total length L in the range of about 2mm to about 4mm, and preferably 3.0mm±0.10mm, and a diameter D in the range of about 100µm to about 200µm, and preferably 150µm±10µm. The support member 11 and the T-shaped fixture members 14A and 14B are made from a suitable biocompatible material for implantation in an eye wall, and preferably stainless steel for enabling slight resilient deformation for facilitating deploying an intraocular drug dispenser 10 in a preferred location in an eye's vitreous cavity.

The drug containing capsules 12 have a biocompatible inert coating 21 and a drug core 22 containing a pharmaceutically active agent which may be in solid, gel or liquid form. The coatings 21 are formed from biocompatible materials, for example, PMMA, and the like, which are impermeable to the passage of the pharmaceutically active agent prior to individual rupturing and which can be ruptured on irradiation with a laser beam causing local heating, local shock waves, and the like.

Figure 2 shows an intraocular drug dispenser 30 including an elongated support member 31 with a string of drug containing capsules 32 having biocompatible inert coatings 33 and drug cores 34 containing a pharmaceutical active agent. The coatings 33 include a throughgoing small diameter bore 36 stopped by a thumbtack-like plug 37 with a shaft portion 38 for sealing a bore 36 and a rounded head 39 formed from suitable biodegradable materials, for example, a solid polymeric matrix formed from derivatised cellulose and methacrylic acid copolymer, and the like. Intraocular drug dispensers 30 are preferably formed with drug containing capsules 32 having plugs 37 of the same material but with heads 39 of different thicknesses for governing the time it takes them to degenerate from the same start time on implantation in an eye. Thus, the plug 37A having the thinnest head is the first plug to degenerate to release its capsule's drug contents whilst the plug 37B having the thickest head is the last plug to degenerate to release its capsule's drug contents.

Alternatively, the drug containing capsules 32 can be stopped by plugs of substantially the same dimensions but formed from different materials having different self-degradation rates for governing their degeneration time from implantation in an eye.

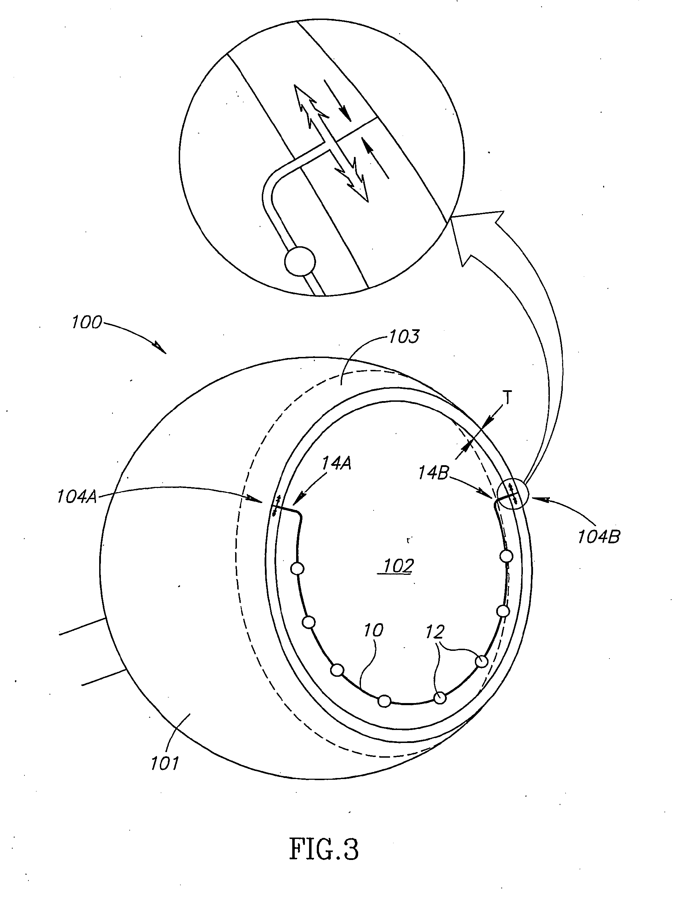

Figure 3 shows an eye 100 having an eye wall 101, and a vitreous cavity 102. The eye wall 101 includes a circumferential pars plana 103 whose width increases from a minimum width W_{N} of about 3.5mm to a maximum width W_{T} of about 4.5mm, and which has a largely uniform thickness T of about 1mm. The implantation of the intraocular drug dispenser 10 in a suitably prepared eye 100 is as follows:

An eye surgeon threads the intraocular drug dispenser 10 with its soft silicone sheath 19 intact from a throughgoing incision 104A made in the pars plana 103 to an opposite throughgoing incision 104B. The eye surgeon removes the soft silicone sheath 19. The eye surgeon inserts the T-shaped fixture member 14A into opposite facing side surfaces of the throughgoing incision 104A so as to draw them together. The eye surgeon bends the support member 11 downwards from the T-shaped fixture member 14A and inserts the T-shaped fixture member 14B into the opposite facing side surfaces of the throughgoing incision 104B so as to draw them together. The support member 11 only marginally disrupts vision, if at all.

In the case of drug containing capsules 12 intended to be ruptured by laser radiation, a physician can employ any standard contact lens for peripheral retina inspection for assisting him in directing a laser beam towards a drug containing capsule for rupturing same. A physician may rupture two drug containing capsules 12 simultaneously to double the drug dosage to a particular patient depending on his clinical condition.

While the invention has been described with respect to a limited number of embodiments, it will be appreciated that many variations, modifications, and other applications of the invention can be made within the scope of the appended claims.

## Claims

1. Intraocular drug dispenser (10; 30) for time controlled release of an intraocular drug in an eye (100) having an eye wall (101) and a vitreous cavity (102), the dispenser comprising an elongated support member (11; 31) having a string of discrete drug containing capsules (12; 32) for individual time controlled release of their drug contents into the eye's vitreous cavity,
wherein said elongated support member has a pair of opposite ends (13) for anchoring into opposite surfaces of the eye's eye wall and a total length of about 30mm±5mm for being urged into a horse shoe shape *in situ* to minimize visual disruption on implantation in the eye.

2. The dispenser according to Claim 1 wherein a drug releasing capsule (12; 32) includes a biocompatible inert coating (21; 33) intended for rupturing on irradiation with a laser beam for releasing its drug contents into the eye's vitreous cavity (102).

3. The dispenser according to Claim 1 wherein a drug releasing capsule (32) includes a biocompatible inert coating (33) with a throughgoing bore (36) for releasing its drug contents into the eye's vitreous cavity (102), and a biodegradable plug (37) for sealing insertion into said bore whereupon self-degradation of said biodegradable plug releases said drug releasing capsule's drug contents into the eye's vitreous cavity.

4. The dispenser according to Claim 3 wherein said string of discrete drug releasing capsules (32) are stopped by biodegradable plugs (37) with substantially the same self-degeneration rates but with different dimensions for time controlled release of their drug contents into the eye's vitreous cavity (102).

5. The dispenser according to Claim 3 wherein said string of discrete drug releasing capsules (32) are stopped by biodegradable plugs (37) with substantially the same dimensions but substantially different self-degradation rates for time controlled release of their drug contents into the eye's vitreous cavity (102).

6. The dispenser according to any one of Claims 1 to 5 wherein each opposite end of said pair of opposite ends (13) is formed with a T-shaped fixation member (14A, 14B) for insertion into opposite facing side surfaces of a throughgoing incision (104A, 104B) formed in the eye's eye wall (101).

7. The dispenser according to Claim 6 wherein a T-shaped member (14A, 14B) subtends an obtuse angle of about 170° with respect to said support member (11).

8. The dispenser according to Claim 7 wherein said each T-shaped fixation member (14A, 14B) includes a pair of oppositely directed self-anchoring anchor members for drawing together opposite facing side surfaces of a throughgoing incision (104A, 104B) formed in the eye's eye wall (101).

9. The dispenser according to any one of Claims 1 to 8 and further comprising a sheath (19) substantially co-extensive with said string of discrete drug containing capsules (12; 32) and enclosing same.

## Patentansprüche

1. Intraokularer Arzneispender (10; 30) zur zeitgesteuerten Freisetzung einer intraokularen Arznei in einem Auge (100), welches eine Augenwand (101) und einen Glaskörperraum (102) hat, wobei der Spender ein längliches Trägerelement (11; 31) umfasst, welches einen Strang von unabhängigen arzneimittelenthaltenden Kapseln (12; 32) zu individuellen zeitgesteuerten Freisetzung ihres Arzneimittelinhalts in den Glaskörper des Auges hat, wobei das längliche Trägerelement ein Paar von gegenüberliegenden Enden (13) zur Verankerung in gegenüberliegenden Flächen der Augenwand des Auges hat und wobei es eine Gesamtlänge von ungefähr 30 mm ± 5 mm hat, um in eine Hufeisenform in situ gebracht zu werden, um eine visuelle Beeinträchtigung bei einer Implantation in dem Auge zu minimieren.

2. Spender nach Anspruch 1, bei dem eine arzneimittelfreisetzende Kapsel (12; 32) eine biokompatible innerte Beschichtung (21; 33) einschließt, welche dazu bestimmt und vorgesehen ist bei einer Bestrahlung mit einem Laserstrahl zu reißen, zur Freisetzung des Arzneimittelinhalts in den Glaskörper (102) des Auges.

3. Spender nach Anspruch 1, bei dem eine arzneimittelfreisetzende Kapsel (32) eine biokompatible innerte Beschichtung (33) einschließt, mit einer Durchgangsbohrung (36) zur Freisetzung ihres Arzneimittelinhalts in den Glaskörper (102) des Auges und einen biologisch abbaubaren Stopfen (37) zur versiegelnden Einsetzung in die Bohrung, wobei die Selbstabbauung des biologisch abbaubaren Stopfens den Arzneimittelinhalt der arzneimittelfreisetzenden Kapsel in den Glaskörper des Auges freisetzt.

4. Spender nach Anspruch 3, bei dem der Strang von unabhängigen arzneimittelfreisetzenden Kapseln (32) durch biologisch abbaubare Stopfen (37) gestoppt ist, mit den im Wesentlichen gleichen Selbstabbauraten aber mit unterschiedlichen Dimensionen, zur zeitgesteuerten Freisetzung ihres Arzneimittelinhalts in den Glaskörper (102) des Auges.

5. Spender nach Anspruch 3, bei dem der Strang von unabhängigen arzneimittelfreisetzenden Kapseln (32) durch biologisch abbaubare Stopfen (37) gestoppt ist, mit den im Wesentlichen gleichen Dimensionen aber mit im Wesentlichen unterschiedlichen Selbstabbauraten, zur zeitgesteuerten Freisetzung ihres Arzneimittelinhalts in den Glaskörper (102) des Auges.

6. Spender nach einem der Ansprüche 1 bis 5, bei dem jedes der gegenüberliegenden Enden des Paares von gegenüberliegenden Enden (13) mit einem T-förmigen Befestigungselement (14A, 14B) geformt ist, zur Einsetzung in gegenüberliegende Seitenflächen einer durchgehenden Incision (104A, 104B), welche in der Augenwand (101) des Auges ausgeformt ist.

7. Spender nach Anspruch 6, bei dem ein T-förmiges Element (14A, 14B) einen stumpfen Winkel von in etwa 170° im Bezug auf das Trägerelement (11) einschießt.

8. Spender nach Anspruch 7, bei dem jedes der T-förmigen Befestigungselemente (14A, 14B) ein Paar von entgegengesetzt ausgerichteten selbstverankemden Ankerelementen einschließt, zum Zusammenziehen von in gegenüberliegende Seitenflächen einer durchgehenden Incision (104A, 104B), welche in der Augenwand (101) des Auges ausgeführt ist.

9. Spender nach einem der Ansprüche 1 bis 8, der weiterhin eine Ummantelung (19) umfasst, die sich im Wesentlichen zusammen mit dem Strang von unabhängigen arzneimittelenthaltenden Kapseln (12; 32) erstreckt und denselben umhüllt.

## Revendications

1. Distributeur de médicament intraoculaire (10; 30), pour une libération commandée dans le temps d'un médicament intraoculaire dans un oeil (100) ayant une paroi d'oeil (101) et une cavité vitreuse (102), le distributeur comprenant un élément support élongé (11 ; 31) ayant une chaîne de capsules distinctes (12 ; 32) contenant le médicament pour une libération individuelle commandée dans le temps de leur contenu de médicament dans la cavité vitreuse de l'oeil,
dans lequel l'élément support élongé a une paire d'extrémités opposées (13) pour ancrage dans des surfaces opposées de la paroi d'oeil de l'oeil, et une longueur totale d'environ 30 mm ± 5 mm, afin d'être contraint dans une forme de fer à cheval, in situ, pour minimiser le bouleversement visuel lors de l'implantation dans l'oeil.

2. Distributeur selon la revendication 1, dans lequel une capsule (12 ; 32) de libération de médicament comprend un revêtement inerte biocompatible (21 ; 33) prévu pour se rompre par irradiation avec un faisceau laser, afin de libérer son contenu de médicament dans la cavité vitreuse de l'oeil (102).

3. Distributeur selon la revendication 1, dans lequel une capsule (32) de libération de médicament comprend un revêtement inerte biocompatible (33) avec un trou traversant (36) pour libérer son contenu de médicament dans la cavité vitreuse d'oeil (102), et un bouchon biodégradable (37) pour étanchéifier l'insertion dans ledit trou, l'auto-dégradation du bouchon biodégradable libérant les contenus de médicament de la capsule de libération de médicament dans la cavité vitreuse de l'oeil.

4. Distributeur selon la revendication 3, dans lequel la chaîne de capsules distinctes de libération de médicament (32) est stoppée par des bouchons biodégradables (37) avec sensiblement les mêmes taux d'auto-dégénérescence, mais avec des dimensions différentes, pour la libération commandée dans le temps de leurs contenus de médicament dans la cavité vitreuse (102) de l'oeil.

5. Distributeur selon la revendication 3, dans lequel la chaîne de capsules (32) distinctes de libération de médicament est stoppée par des bouchons biodégradables (37), ayant sensiblement les mêmes dimensions mais des taux sensiblement différents d'auto-dégradation, pour une libération commandée dans le temps de leurs contenus de médicament dans la cavité vitreuse (102) de l'oeil.

6. Distributeur selon l'une quelconque des revendications 1 à 5, dans lequel chaque extrémité opposée de la paire d'extrémités opposées (13) est formée avec un élément de fixation en forme de T (14A, 14B), pour une insertion dans des surfaces latérales opposées en regard d'une incision traversante (104A, 104B) formée dans la paroi (101) d'oeil de l'oeil.

7. Distributeur selon la revendication 6, dans lequel un élément en forme de T (14A, 14B) sous-tend un angle obtus d'environ 170 degrés par rapport à l'élément support (11).

8. Distributeur selon la revendication 7, dans lequel chaque élément de fixation en forme de T (14A, 14B) comprend une paire d'éléments d'ancrage auto-ancrant orientés de manière opposée, afin de dessiner ensemble des surfaces latérales opposées en regard d'une incision traversante (104A, 104B) formée dans la paroi (101) d'oeil de l'oeil.

9. Distributeur selon l'une quelconque des revendications 1 à 8, et comprenant, en outre, une gaine (19) coextensive avec la chaîne de capsules distinctes (12 ; 32) contenant le médicament et englobant celle-ci.
